# EUROPEAN PATENT SPECIFICATION

(11) **EP 3 082 719 B1**
(45) Date of publication and mention of the grant of the patent: **23.03.2022**
(21) Application number: 14815332.3
(22) Date of filing: 17.12.2014
(51) Int. Cl.: A61K 8/26, A61Q 11/00, A61K 8/02

(54) **DENTIFRICE COMPOSITION COMPRISING SPHERICAL FUSED ALUMNIUM OXIDE PARTICLES**
ZAHNPASTAZUSAMMENSETZUNG MIT KUGELFÖRMIGEN UND KONDENSIERTEN ALUMINIUMOXIDTEILCHEN
COMPOSITION DE DENTIFRICE COMPRENANT DES PARTICULES D'OXYDE D'ALUMINIUM FUSIONNÉES SPHÉRIQUES

(30) Priority: 19.12.2013 GB 201322510
(43) Date of publication of application: 26.10.2016
(73) Proprietor: GlaxoSmithKline Consumer Healthcare (UK) IP Limited, Brentford, Middlesex TW8 9GS (GB)
(72) Inventor: LUCAS, Robert Anthony, Weybridge Surrey KT13 0DE (GB)
(74) Representative: Lubienski, Michael John
(86) International application number: PCT/EP2014/078130
(87) International publication number: WO 2015/091594

(56) References cited:
- JP-A- 2001 104 469
- US-A- 3 060 098
- US-A1- 2012 219 606
- US-B1- 6 685 918

## Description

The present invention relates to dentifrice compositions comprising spherical fused aluminium oxide particles as an abrasive agent and an orally acceptable carrier. Such compositions can effectively clean, polish and remove stains from the surface of teeth or dentures without a high degree of abrasion thereby reducing scratching and damage to the tooth or denture surface. Such compositions thereby provide superior cleaning, polishing, gentle stain removal and whitening of tooth surfaces or dentures.

Oral care compositions, in particular dentifrices, are used to clean teeth on a daily basis. Dentifrices will aid in the removal of food particles and tooth discolouration caused by substances such as tobacco, tea or wine in addition to the removal of plaque and other soft materials from the tooth. Cleaning and polishing the tooth surfaces is effected by either chemical processes such as materials that bind to elements within plaque or by mechanical means such as abrasive substances.

Dentifrices commonly incorporate an abrasive material for mechanical cleaning and polishing of teeth by abrading deposits. The abrasive material is primarily intended to effect mechanical removal of deposits from the surface of teeth, e.g. by removal of the pellicle film and plaque adhered to the tooth surface. The plaque and pellicle film is prone to discolouration and staining for example by comestibles such as tea and coffee and by tobacco, resulting in unsightly appearance of the teeth. While such mechanical removal is important for effecting cleaning, it is vital that the abrasive used is not unduly harsh in order to minimise damage to the tooth surface. In addition to cleaning and polishing the teeth, white teeth have long been considered cosmetically desirable.

US 6,517,815 (Henkel Kommanditgesellschaft auf Aktien) discloses a dentifrice in the form of an aqueous paste or liquid dispersion, comprising 10% to 30% by weight of a combination of silica polishing agents and aluminium oxide (Alumina) in a ratio by weight of 10:0.2 to 10:2, 20% to 50% by weight of a humectants and 2% to 12% by weight of a condensed phosphate. The condensed phosphates are in the form of an alkali metal or ammonium salt. The aluminium oxide is preferably a lightly calcined alumina with a content of at least 10% by weight of α-aluminium oxide of various γ- aluminium oxides. It is suggested that the special combination of polishing agents (i.e. the combination of the silica and the alumina) is able to provide a dentifrice having good polishing and cleaning effects with only moderate dentine and enamel abrasion, notwithstanding the presence of the hard alumina polishing component.

US 4,632,826 (Henkel Kommanditgesellschaft auf Aktien) discloses a tooth cream comprising 100 parts by weight of silica polishing agent and 2 to 15 parts by weight of weakly calcined alumina polishing agent. The silica polishing agent consists essentially of silica hydrogel and precipitated silica and the weakly calcined alumina 10 to 50% by weight of γ-aluminium oxide and 50 to 90% by weight of α-aluminium oxide. The tooth cream is suitable for removing stain, polishing and cleaning the surface of teeth without producing any deep scratches or damage by daily use.

Klüppel et al. J. Soc. Cosmet. Chem., 37, 211-223 (July/August 1986) "Parameters for assessing the cleaning power of toothpastes" compares a number of dentifrice formulations for polishing and scratching effects. Test formulations are described containing as the sole abrasive material either a polishing alumina or hydrated silica or a mixture of a hydrated silica with a polishing alumina. The results suggest that dentifrice products can be developed with high cleaning power and low dentine abrasion. Whilst the test formulations with increasing amounts of a polishing alumina, as the sole abrasive, can provide good cleaning power this was coupled with an unacceptable increase in enamel abrasion. The best test formulation contained a mixture of a silica abrasive and an alumina abrasive which exhibited a high cleaning value together with an unexpectedly low enamel abrasion value.

WO2010/068433, WO 2010/068471, US2012/0219606 and related patent applications all claiming priority from US provisional application 61/117,856 (Procter and Gamble) describe oral care compositions comprising fused silica particles, which may be spherical (e.g. at least 25% or at least 95% of the particles may be spherical). These documents indicate that the shape of fused silica particles may be angular or spherical depending upon the type of the manufacturing process, which shape can impact upon the abrasivity of the fused silica. For example, at the same particle size, spherical fused silica may have a lower radioactive dentin abrasion (RDA) than that of angular fused silica. Consequently, it may be possible to optimize cleaning capability whilst not increasing abrasivity. It is further stated in these documents that compositions that comprise such spherical fused silica have certain advantages: due to the rounded edges, the spherical fused silica may be less abrasive. This means that the PCR to RDA ratio can be improved while still providing good cleaning. Also spherical fused silica may be used at higher levels without being too abrasive. The spherical fused silica may also be used in combination with the angular fused silica, or silica wherein at least about 25% of the particles are angular, to help lower costs, while still delivering good cleaning with acceptable abrasivity.

WO2011/120943 (Glaxo Group Limited), discloses a dentifrice compositions comprising calcined aluminium oxide polishing agent and a condensed phosphate (such as a water soluble alkali metal polyphosphate salt) which, in the absence of any silica abrasive material, can effectively whiten and polish the enamel of teeth and the surface of dental prostheses without a high degree of abrasion and scratching of the enamel surface.

US 6,083,489 (Ultradent Products Inc), discloses toothpastes and other dentifrices formulated to include substantially spherical cleaning particles for enhanced plaque removal capability. Preferred cleaning particles include amongst others hollow aluminium oxide spheres with a particle size in a range from about 10 microns to about 200 microns. The larger, more spherical cleaning particles are said to be far less abrasive but have greatly enhanced plaque removal power compared to conventional abrasives and polishes used in toothpastes. The compositions of US 6,083,489 are said to be effective for removing plaque and other soft tooth deposits but may not be as effective in removing tenacious stains adhered to the tooth surface.

US3060098 discloses a dentifrice capable of givine a high luster to teeth and more particularly to a dentifrice having high luster-producing characteristics comprising α-alumina in admixture with a polishing agent having a Mohs hardness below about 6.

JP2001/104469 discloses alumina powder obtained by heating normal alpha alumina powder to 1500°C or more, preferably within a sudden heating being carried out at 2000°C or more so that the surface fuse/melts and becomes spherical due to the surface tension.

US6685918 discloses an oral cleansing product comprising as an abrasive an alumnina in the form of particles having d₁₀ below 3.5 µm, d₅₀ below 1.0 µm, and a specific surface area below 6 m²/g.

There remains a need for compositions with improved cleaning to remove both soft tooth deposits, such as plaque and hard deposits, such as stains and tartar, without increased scrubbing and abrading of the tooth surface.

It has now been found that spherical fused aluminium oxide when used in dentifrice compositions delivers effective cleaning, polishing, stain removal and whitening whilst at the same time providing low tooth abrasivity to the tooth or denture surface.

Accordingly in a first aspect the present invention provides a dentifrice composition comprising spherical fused aluminium oxide particles, as defined in claim 1, as an abrasive and an orally acceptable carrier.

Compositions of the present invention provide good cleaning and polishing of the teeth without the use of harsh abrasives.

Spherical aluminium oxide is currently used for its high sphericity (that results in high flowability and pack density), surface hardness, high thermal conductivity and heat resistance in the field of electronic materials.

Spherical fused aluminium oxide may be prepared from angular powdered alumina and passing it through a fusing process that involves raising the temperature to greater than 2000°C, which melts the particle surface, the resulting surface tension produces a sphere. The particle is then cooled, before classifying and sieving to isolate the particle size distribution of interest.

For the avoidance of doubt, spherical fused aluminium oxide particles include any particle where the whole particle is mostly rounded or elliptical in shape.

Suitably at least 90% of the spherical fused aluminium oxide particles are essentially spherical, more suitably 95% of the spherical fused aluminium oxide particles are essentially spherical, even more suitably 99% of the spherical fused aluminium oxide particles are essentially spherical.

The spherical fused aluminium oxide particles used in the present invention may be obtained from suitable sources such as Denka Chemical GmbH, Nippon Steel & Sumikin Materials Co Ltd and Micron Co.

The spherical fused aluminium oxide particles have a median particle size, (as determined by laser diffraction), in the range from about 1 to about 15 microns with a maximum span of about 2.5. More suitably the particles have a median particle size in the range from about 2 to 10 microns with a maximum span of about 2.0 or below.

An example of such a spherical fused aluminium oxide may be AX3-10 from Mircon Co., with a D 0.1 of 1.26 microns, D 0.5 of 4.53 microns and a D 0.9 of 9.82 microns.

Span describes the width of the distribution based on the 0.1, 0.5 and 0.9 quantile, and is expressed as (D 0.9 - D 0.1)/D 0.5. The D 0.1 value is where 10% of the particles are below this value, the D 0.5 value is the median diameter where 50% of the distribution is above this value and 50% is below this value and the D 0.9 value is where 90% of the particles are below this value.

Suitably the particle distribution is monomodal. By monomodal is meant a particle size distribution that has a single mode, whereby a mode of a discrete distribution is a value at which the amount of particles (typically measured by volume or mass) according to their size takes its maximum value.

The spherical fused aluminium oxide particles are present in an amount from about 0.1% to about 1.0% by weight of the total composition..

A dentifrice composition according to the invention may further comprise a supplementary abrasive agent, provided that such agent(s) do(es) not significantly adversely impact on dentine abrasion. Suitable examples of supplementary abrasive agents for use in the present invention include silica, alumina, hydrated alumina, calcined alumina, calcium carbonate, anhydrous dicalcium phosphate, dicalcium phosphate dihydrate, water-insoluble sodium metaphosphate, zirconia, perlite, diamond, rice hull silica, silica gels, aluminium silicates, pyrophosphates, pumice, polymer particles, calcium phosphate based minerals (e.g. tricalcium phosphate (TCP), hydrated HA and mixed phase (HA:TCP) calcium phosphate mineral) and/or any other whitening agent and mixtures thereof. A supplementary abrasive agent may be used generally in an amount ranging from about 0.1%w/w to about 50%w/w or about 0.1%w/w to about 20%w/w by weight of the total dentifrice composition.

In one aspect of the present invention spherical fused aluminium oxide is the sole abrasive agent.

Radioactive dentine abrasion (RDA) is a measure of the abrasiveness of a dentifrice. The established method for determining the abrasivity of a dentifrice formulation is by measuring the Relative Dentine Abrasivity (RDA) (Hefferen, JJ. A laboratory method for measuring dentifrice abrasivity. J. Dent. Res. 55 563-573, 1976.). This assay measures loss of dentine due to extended brushing with a 25:40 w/w slurry of test material, e.g. toothpaste, from prepared samples of human dentine. The dentine samples are irradiated to generate ³²P in the mineral. The assay measures radioactivity in the supernatant after brushing, relative to radioactivity liberated by brushing with a standard slurry of calcium pyrophosphate.

Advantageously compositions of the present invention will comprise a low RDA value ranging from about 10 to about 100, suitably in the range from about 20 to about 80.

The cleaning ability of dentifrices may be demonstrated by using the Pellicle Cleaning Ratio (PCR) test - a laboratory method accepted as useful in the characterization of stain cleaning (whitening) actions of abrasive-containing dentifrices. The PCR value is calculated relative to a standard material (Ca₂P₂O₇, Odontex Inc.) which is given the empirical value of 100.

Advantageously a dentifrice according to the present invention will comprise a PCR value ranging from about 50 to about 130, suitably in the range from about 60 to about 120.

Surprisingly the spherical fused aluminium oxide for use in the present invention has been shown to provide excellent cleaning at low concentrations.

A dentifrice according to the present invention shows excellent cleaning and stain removal of the tooth surface with minimal dentine abrasion. The high cleaning/low abrasivity properties of a dentifrice according to the invention may also be reflected in the Cleaning Efficiency Index value for the dentifrice. The Cleaning Efficiency Index value can be readily determined by one skilled in the art. See Schemehorn BR, Ball TL, Henry GM, Stookey GK. "Comparing dentifrice abrasive systems with regard to abrasion and cleaning." J. Dent Res 1992; 71: 559.

Advantageously a dentifrice according to the present invention will comprise a CEI value ranging from about 1.25 to about 2.6, suitably in the range from about 1.4 to about 2.4.

Radioactive enamel abrasion (REA) is another measure of the abrasiveness of a dentifrice. The established method for determining the abrasivity of a dentifrice formulation is by measuring the Relative Enamel Abrasivity (REA) (Hefferen, JJ. A laboratory method for measuring dentifrice abrasivity. J. Dent. Res. 55 563-573, 1976.). This assay measures loss of enamel due to extended brushing with a 25:40 w/w slurry of test material, e.g. toothpaste, from prepared samples of human enamel. The enamel samples are irradiated to generate ³²P in the mineral. The assay measures radioactivity in the supernatant after brushing, relative to radioactivity liberated by brushing with a standard slurry of calcium pyrophosphate.

Advantageously a dentifrice according to the present invention will comprise an REA value of about 15 or below, where the maximum safe and allowable REA value is 40.

A dentifrice composition of the present invention can therefore provide good cleaning with low abrasivity, resulting in cleaner, whiter and highly polished tooth surfaces, with less or no staining, reduced plaque and tartar resulting in improved oral health.

A dentifrice composition of the present invention may further comprise a water-soluble condensed phosphate salt, such as an alkali metal pyrophosphate, tripolyphosphate or higher polyphosphate salt, in particular a water soluble alkali metal tripolyphosphate salt. Suitably the sodium form of this salt is preferred, although the potassium or mixed sodium and potassium salts could be used as a preferred embodiment as well. All physical forms can be used, e.g. a hydrate or the dehydrated form.

Most suitably the water soluble alkali metal tripolyphosphate salt is sodium tripolyphosphate.

Suitably the water soluble condensed phosphate salt (such as an alkali metal tripolyphosphate salt) is present in an amount from 1.0% to 20.0%, for example from 2.0% to 15.0% or 5.0% to 10.0% by weight of the total composition.

A dentifrice composition of the present invention may comprise one or more active agents conventionally used in dentifrice compositions, for example, a fluoride source, a desensitising agent, an anti-bacterial agent, an anti-plaque agent, an anti-calculus agent, an oral malodour agent, an anti-inflammatory agent, an anti-oxidant, an anti-fungal agent, wound healing agent or a mixture of at least two thereof. Such agents may be included at levels to provide the desired therapeutic effect.

Examples of desensitising agents include a tubule blocking agent or a nerve desensitising agent and mixtures thereof, for example as described in WO02/15809 (Block). Examples of desensitising agents include a strontium salt such as strontium chloride, strontium acetate or strontium nitrate or a potassium salt such as potassium citrate, potassium chloride, potassium bicarbonate, potassium gluconate and especially potassium nitrate.

A desensitising agent such as a potassium salt is generally present between 2% to 8% by weight of the total composition, for example 5% by weight of potassium nitrate may be used.

In one embodiment the desensitizing agent comprises a bioactive glass. Suitably the bioactive glass consists of about 45% by weight silicon dioxide, about 24.5% by weight sodium oxide, about 6% by weight phosphorus oxide, and about 24.5% by weight calcium oxide. One such bioactive glass is available commercially under the trade name, NovaMin^{®}, also known as 45S5 Bioglass^{®}.

Suitably the bioactive glass is present in an amount ranging from about 1% to about 20% by weight of the dentifrice, such as from about 1% to about 15%, or such as from about 1% to about 10%, or such as from about 2% to about 8 % by weight of the dentifrice composition.

In another embodiment the desensitizing agent comprises an arginine calcium carbonate salt. Suitably the arginine salt is present in an amount ranging from about 0.5%w/w to 30%w/w of the dentifrice, such as from about 1% to 10%w/w, or such as from about 1% to about 10%w/w, or such as from about 2% to about 8%w/w of the dentifrice composition.

In a further embodiment the desensitizing agent includes a tubule blocking agent, such as a silica, colloidal silica, nano zinc oxide, sub-micron alumina and sub micron polymer beads, in a fine particulate form comprising an average particle size in the range from about 1nm to about 5microns.

Suitable sources of fluoride ions for use in the compositions of the present invention include an alkali metal fluoride such as sodium fluoride, an alkali metal monofluorophosphate such a sodium monofluorophosphate, stannous fluoride, or an amine fluoride in an amount to provide from 25 to 3500ppm of fluoride ions, preferably from 100 to 1500ppm. A typical fluoride source is sodium fluoride, for example the composition may contain 0.1 to 0.5% by weight of sodium fluoride, e.g. 0.204% by weight (equating to 923 ppm of fluoride ions), 0.2542% by weight (equating to 1150ppm of fluoride ions) or 0.315% by weight (equating to 1426ppm of fluoride ions).

Such fluoride ions help promote the remineralisation of teeth and can increase the acid resistance of dental hard tissues for combating caries, dental erosion (ie. acid wear) and/or tooth wear.

Compositions of the present invention will contain additional formulating agents such as, surfactants, humectants, non-abrasive (thickening) silicas, flavouring agents, sweetening agents, opacifying or colouring agents, preservatives and water, selected from those conventionally used in the oral hygiene composition art for such purposes.

Suitable surfactants for use in the present invention include anionic surfactants such as a sodium C₁₀₋₁₈alkyl sulphate, e.g. sodium lauryl sulphate. Sodium lauryl sulphate is generally considered to be anionic and strongly charged and is useful if high levels of foaming are desired when brushing teeth.

In addition to anionic surfactants, zwitterionic, amphoteric, cationic and non- or low-ionic surfactants may be used to aid foaming characteristics. When anionic and amphoteric surfactants are used together an optimised foaming system is achieved that will provide both improved mouth feel and good cleaning. Examples of amphoteric surfactants include long chain alkyl (e.g. C₁₀-C₁₈ alkyl) betaines, such as the product marketed under the trade name 'Empigen BB' by Albright & Wilson and long chain alkyl amidoalkyl betaines such as cocamidopropylbetaine.

A particularly preferred example of an anionic/amphoteric surfactant combination for use in the present invention is sodium lauryl sulphate/cocamidopropylbetaine.

Suitably, the surfactant is present in the range from about 0.1 to about 15%, for example from about 0.5 to about 10% or from about 1.0 to about 5% by weight of the total composition.

Suitable humectants for use in compositions of the invention include glycerin, xylitol, sorbitol, propylene glycol or polyethylene glycol, or mixtures of at least two thereof; which humectant may be present in the range from about 10 to about 80%, for example from about 20 to about 70% or from about 30 to about 60% by weight of the total composition.

It will be understood that compositions of the present invention may also be used outside the oral cavity, for the cleaning of dentures and the like.

The dentifrice compositions of the present invention are typically formulated in the form of toothpastes, instant powders, tablets and gels. Preferred compositions of the present invention are toothpastes and gels.

The dentifrices of the present invention are typically formulated in the form of a paste that is suitable for containing in and dispensing from a laminate tube or a pump as conventionally used in the art. Additional examples may include bag-in-can or bag-on-valve delivery systems that utilise a foaming agent such as pentane or iso-pentane.

A typical process for making the composition of this invention involves admixing the ingredients, suitably under a vacuum, until a homogeneous mixture is obtained, and adjusting the pH if necessary.

The invention is further illustrated by the following examples.

### Examples 1 - 4

**Table 1.**

| **Ingredient Name** | **Example 1** | **Example 2** | **Example 3** | **Example 4** |
|---|---|---|---|---|
| | **Qty (% w/w)** | **Qty (% w/w)** | **Qty (% w/w)** | **Qty (% w/w)** |
| Sorbitol Liquid [NC] | 28.500 | 28.500 | 28.500 | 28.500 |
| PEG 300 (PEG-6) | 3.000 | 3.000 | 3.000 | 3.000 |
| Glycerol | 7.600 | 7.600 | 7.600 | 7.600 |
| Denka Spherical alumina DAW-03 | 0.100 | 0.250 | 0.500 | |
| Sanyo Spherical alumina AX3-10R | | | | 0.250 |
| Zeofree 153b | 16.400 | 16.250 | 16.000 | 16.250 |
| Tegobetain CKD | 0.600 | 0.600 | 0.600 | 0.600 |
| Adinol CT95 | 0.600 | 0.600 | 0.600 | 0.600 |
| Xanthan Gum | 0.800 | 0.800 | 0.800 | 0.800 |
| Genevisco TPH-1 [U0931] | 0.400 | 0.400 | 0.400 | 0.400 |
| Titanium Dioxide | 0.600 | 0.600 | 0.600 | 0.600 |
| Potassium Nitrate | 5.000 | 5.000 | 5.000 | 5.000 |
| Saccharin Sodium | 0.300 | 0.300 | 0.300 | 0.300 |
| Spearmint oil, American | 0.500 | 0.500 | 0.500 | 0.500 |
| Peppermint oil American / Flavour Mac 49 Peppermint Oil, JCIC | 0.500 | 0.500 | 0.500 | 0.500 |
| Sucralose Powder | 0.050 | 0.050 | 0.050 | 0.050 |
| Sodium Fluoride | 0.3152 | 0.3152 | 0.3152 | 0.3152 |
| Sodium Hydroxide | 0.080 | 0.080 | 0.080 | 0.080 |
| P. Water | 34.6548 | 34.6548 | 34.6548 | 34.6548 |
| Total | 100.000 | 100.000 | 100.000 | 100.000 |

Test data was obtained for the formulations listed in table 1. The formulations were prepared by admixing the ingredients listed above in the following order.
1. Mix glycerol and sorbitol
2. Add abrasive
3. Add KNO₃, TiO₂, saccharin, sucrolose, sodium fluoride and sodium hydroxide
4. Add thickening silica
5. Add surfactants
6. Add gums
7. Add flavour oils.

### RDA methodology

### Specimen Preparation

The procedure used in this study was the Hefferren abrasivity test recommended by the ADA and ISO 11609 for determination of dentifrice relative abrasiveness in dentin.

Eight (8) human dentin specimens were subjected to neutron bombardments resulting in the formation of radioactive phosphorus (³²P) within the specimens under the controlled conditions outlined by the ADA. The specimens were then mounted in methyl methacrylate so they would fit in a V-8 cross-brushing machine. The specimens were preconditioned by brushing for 1500-strokes, (soft Oral B-40; 150g brush tension) using a slurry consisting of 10g ADA reference material in 50 ml of a 0.5% CMC glycerin solution.

### Procedure

Following the precondition run, the test was performed (150g and 1500 strokes) using in a "sandwich design". Before and after being brushed with the dentifrice slurry (25g/40ml water) each tooth set was brushed with the ADA reference material (10g of Ca₂P₂O₇/50 ml0.5% CMC). The procedure was repeated several times so that each product was assayed on each tooth set. The treatment design was a modified Latin Square design so that no treatment followed another treatment consistently.

### Calculations

One ml samples were taken, weighed (~1g), and added to 5 ml of "*Ultima Gold*" scintillation cocktail. The samples were mixed well and immediately put on the scintillation counter for radiation count. Following counting, the net counts per minute (CPM) values were divided by the weight of the sample to calculate the net CPM/gram per slurry. The net CPM/g of the pre and post ADA reference material for each of the test slurries was then calculated and averaged to use in the calculation of RDA (relative dentin abrasion) for the test material. The ADA reference material was assigned a value of 100 and its ratio to the test material was calculated.

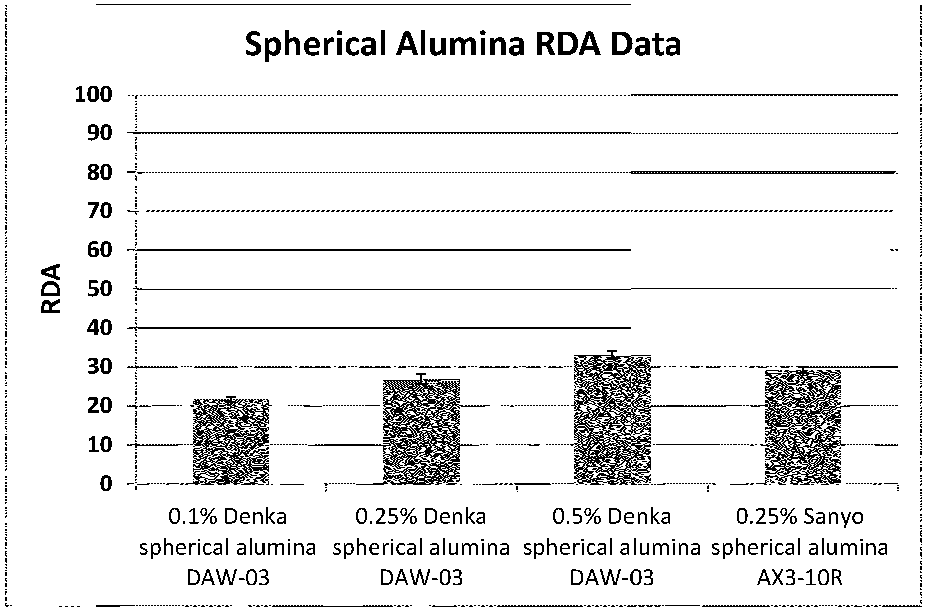

The results demonstrate that at low concentrations spherical fused aluminium oxide provides low levels in dentine abrasivity .

### Example 5

### PCR Methodology

### Specimen Preparation

Bovine, permanent, central incisors were cut to obtain labial enamel specimens approximately 10 X 10 mm. The enamel specimens were then embedded in an autopolymerizing methacrylate resin so that only the enamel surfaces were exposed. The enamel surfaces were then smoothed and polished on a lapidary wheel and lightly etched to expedite stain accumulation and adherence. They were placed on a rotating rod (in 37°C incubator) alternately exposing them to air and to a solution consisting of trypticase soy broth, tea, coffee, mucin, FeCl3, and *Micrococcus luteus* BA13. The staining broth was changed and specimens rinsed daily for seven days. After seven days, a darkly stained pellicle film was apparent on the enamel surfaces. Specimens were then rinsed, allowed to air dry, and refrigerated until use. All products were tested using specimens prepared at the same time.

### Scoring and Set-Up

The amount of *in vitro* stain was graded photometrically using only the L value of the L^{∗}a^{∗}b^{∗} scale using a spectrophotometer (Minolta CM2600d.). The area of the specimens scored was a 1/4-inch diameter circle in the centre of the 10 x 10 mm enamel. Specimens with scores between 30 and 42 (30 being more darkly stained) were used. On the basis of these scores, the specimens were divided into groups of 16 specimens each, with each group having the same average baseline score.

### Test Procedure

The specimens were then mounted on a mechanical V-8 cross-brushing machine equipped with soft nylon-filament (Oral-B 40) toothbrushes. Brush force on the enamel surface was adjusted to 150 g. The dentifrices were used as slurries prepared by mixing 25 grams of dentifrice with 40 ml of deionized water. The ADA abrasion reference material (Ca₂P₂O₇) was prepared by mixing 10 g in 50 ml of a 0.5% CMC solution. The specimens were brushed for 800 strokes (4 1/2 minutes). To minimize mechanical variables, one specimen per group was brushed on each of the eight brushing heads. Fresh slurries were made after being used to brush four specimens. Following brushing, specimens were rinsed, blotted dry, and scored again for stain as previously described.

### Calculations

The difference between the pre- and post-brushing stain scores was determined and the mean and standard error calculated for the reference group. The cleaning ratio for the reference material group was assigned a value of 100. The mean decrement of the reference group was divided into 100 to obtain a constant value to multiple times each individual test decrement within the study. The individual cleaning ratio of each specimen was then calculated (decrement X constant). The mean and SEM for each group (N=16) was then calculated using the individual cleaning ratios. The larger the value of the cleaning ratio, the greater the amount of stained pellicle removed in this test.

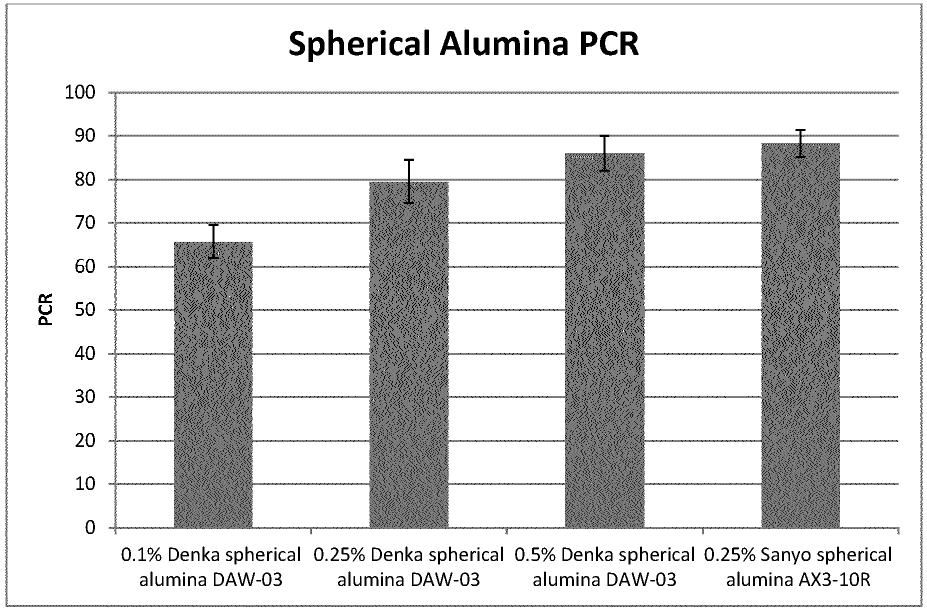

The results demonstrate that at low concentrations spherical fused aluminium oxide provides good levels of cleaning.

### Example 6

### REA Methodology

### Specimen Preparation

Eight (8) human enamel specimens were subjected to neutron bombardments resulting in the formation of radioactive phosphorus (³²P) within the specimens under the controlled conditions outlined by the ADA. The specimens were mounted in methyl methacrylate so they fit in a V-8 cross-brushing machine. The specimens were brushed for a 5000 stroke, precondition run using slurry consisting of 10g ADA reference material in 50 ml of a 0.5% CMC glycerin solution. The brushes used were those specified by the ADA with a brush tension of 150g.

### Procedure

Following the precondition run, the test was performed using the above parameters (150g and 5000 strokes) in a "sandwich design." Before and after brushing with the test product (25 g product/40 ml water) each tooth set was brushed with the ADA Reference Material (10g of Ca2P2O7/50ml 0.5% CMC). The procedure was repeated several times so that each product was assayed on each tooth set. The treatment design was the modified Latin Square design so that no treatment followed another treatment consistently.

### Calculations

One ml samples were taken, weighed (~1g), and added to 5 ml of "Ultima Gold' scintillation cocktail. The samples were mixed well and immediately put on a liquid scintillation counter for radiation detection. Following counting, the net counts per minute (CPM) values were divided by the weight of the sample to calculate a net CPM/gram of slurry. The net CPM/g of the pre and post ADA reference material for each of the test slurries was calculated and averaged to use in the calculation of REA (relative enamel abrasion) for the test material. The ADA material was assigned a value of 10 and its ratio to the test material calculated.

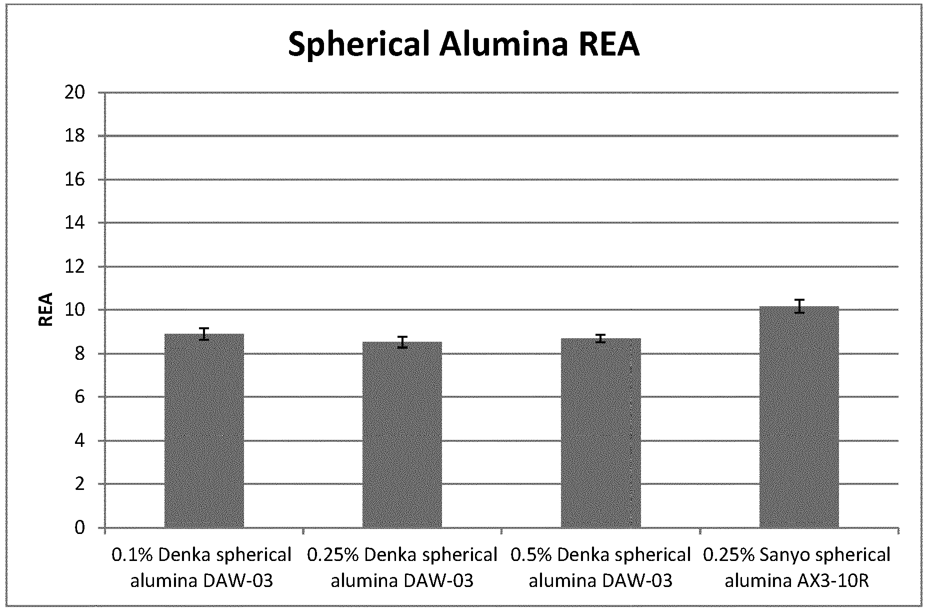

The results demonstrate that at low concentrations spherical fused aluminium oxide provides low enamel abrasivity.

### Example 7

### CEI Data

The values obtained in the above PCR and RDA experiments were then used to obtain the following Cleaning Efficiency Index (CEI) which is the (PCR - 50 + RDA) /RDA value.

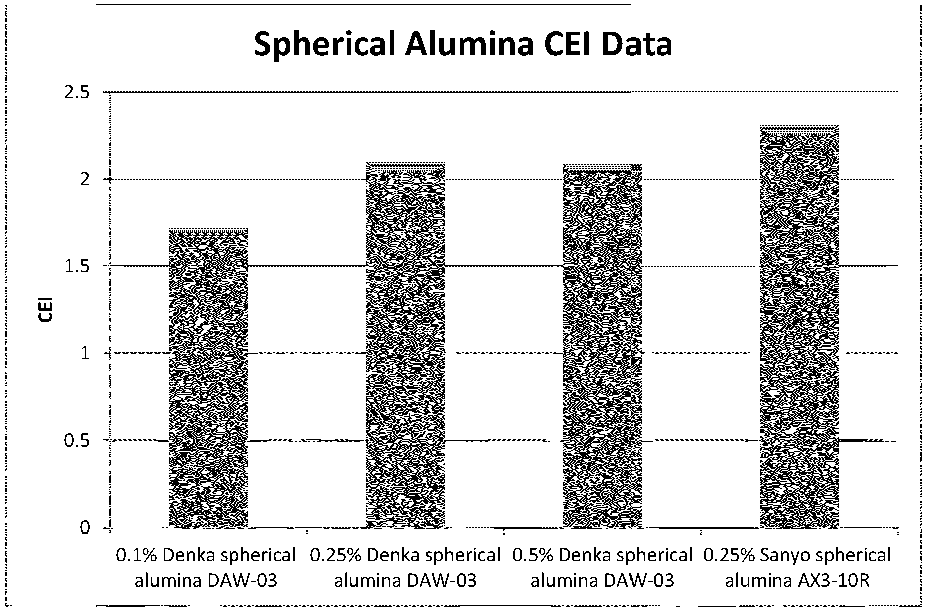

The results demonstrate that at low concentrations spherical fused aluminium oxide provides efficient cleaning.

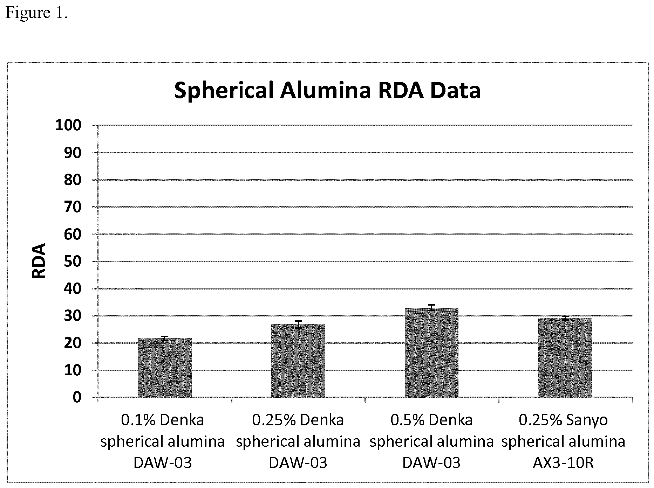

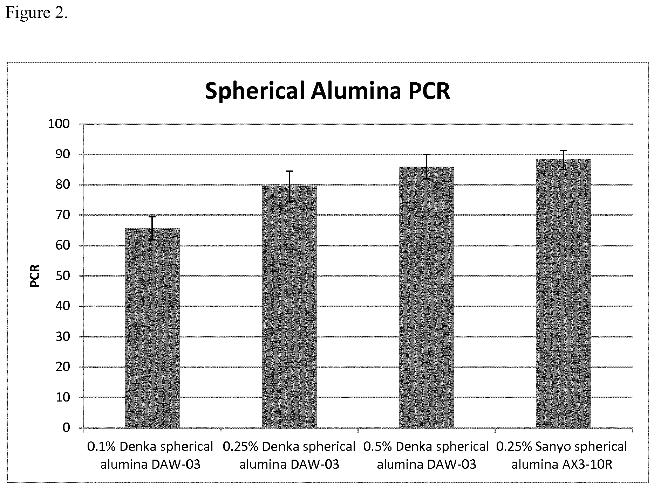

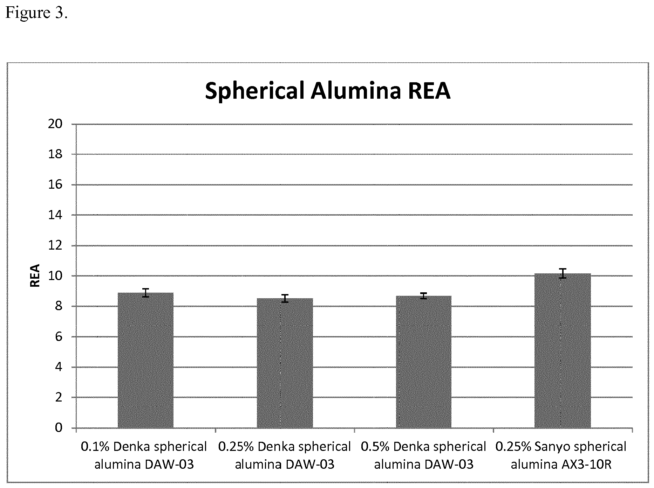

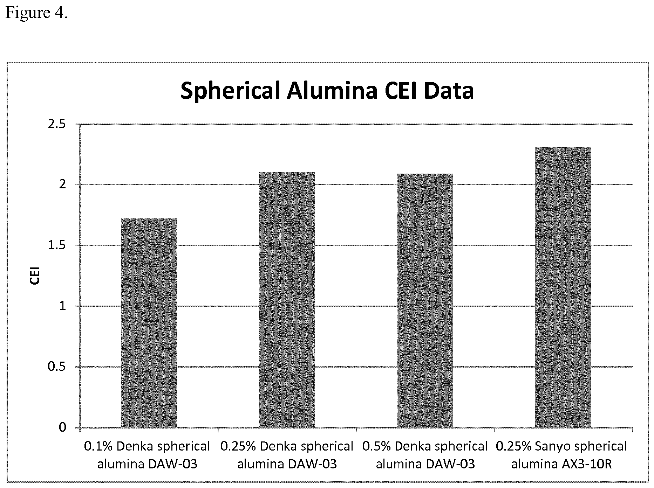

## Claims

1. A dentifrice composition comprising spherical fused aluminium oxide particles as an abrasive in an amount from 0.01% to 1.0% by weight of the composition and wherein the median particle size of the spherical fused aluminium oxide particles is in the range from 1 to 15 microns, as determined by laser diffraction, with a maximum span of about 2.5, wherein span describes the width of the distribution based on the 0.1, 0.5 and 0.9 quantile, and is expressed as (D 0.9 - D 0.1)/D 0.5; the D 0.1 value is where 10% of the particles are below this value, the D 0.5 value is the median diameter where 50% of the distribution is above this value and 50% is below this value and the D 0.9 value is where 90% of the particles are below this value;
and an orally acceptable carrier.

2. A composition as claimed in claim 1 comprising a supplementary abrasive agent.

3. A composition as claimed in any one of claims 1 or 2 wherein the spherical fused aluminium oxide is the sole abrasive in the dentifrice.

4. A composition as claimed in any one of claims 1 to 3 comprising a water soluble condensed phosphate.

5. A composition as claimed in claim 4 wherein the condensed phosphate is a water soluble alkali metal tripolyphospate salt.

6. A composition as claimed in any one of claims 1 to 5 comprising a desensitizing agent.

7. A composition as claimed in any one of claims 1 to 6 comprising a source of fluoride ions.

## Patentansprüche

1. Zahnpastazusammensetzung, umfassend kugelförmige, verschmolzene Aluminiumoxidteilchen als ein Schleifmittel in einer Menge von 0,01 bis 1,0 Gew.-% der Zusammensetzung, wobei die mittlere Teilchengröße der kugelförmigen, verschmolzenen Aluminiumoxidteilchen im Bereich von 1 bis 15 Mikrometer liegt, bestimmt durch Laserbeugung, mit einer maximalen Spannweite von ungefähr 2,5, wobei die Spannweite die Breite der Verteilung basierend auf dem 0,1-fachen, 0,5-fachen und 0,9-fachen Quantil beschreibt und ausgedrückt wird als (D 0,9 - D 0,1)/D 0,5; worin der D 0,1 Wert bezeichnet, dass 10% der Teilchen unter diesem Wert liegen, der D 0,5 Wert der mittlere Durchmesser ist, bei dem 50% der Verteilung über diesem Wert liegt und 50% der Verteilung unter diesem Wert liegt, und der D 0,9 Wert bezeichnet, dass 90% der Teilchen unter diesem Wert liegen;
und einen oral akzeptablen Träger.

2. Zusammensetzung nach Anspruch 1, die ein ergänzendes Schleifmittel umfasst.

3. Zusammensetzung nach Anspruch 1 oder 2, wobei das kugelförmige, verschmolzene Aluminiumoxid das einzige Schleifmittel in der Zahnpasta ist.

4. Zusammensetzung nach mindestens einem der Ansprüche 1 bis 3, die ein wasserlösliches kondensiertes Phosphat umfasst.

5. Zusammensetzung nach Anspruch 4, wobei das kondensierte Phosphat ein wasserlösliches Alkalimetall-Tripolyphosphatsalz ist.

6. Zusammensetzung nach mindestens einem der Ansprüche 1 bis 5, die ein Desensibilisierungsmittel umfasst.

7. Zusammensetzung nach mindestens einem der Ansprüche 1 bis 6, die eine Fluoridionenquelle umfasst.

## Revendications

1. Composition de dentifrice comprenant des particules d'oxyde d'aluminium fusionné sphérique en tant qu'abrasif en une quantité de 0,01 % à 1,0 % en poids de la composition et dans laquelle la taille particulaire médiane des particules d'oxyde d'aluminium fusionné sphérique est dans la plage de 1 à 15 microns, telle que déterminée par diffraction laser, avec un intervalle maximal d'environ 2,5, dans laquelle l'intervalle décrit la largeur de la distribution sur la base des quantiles 0,1, 0,5 et 0,9, et est exprimé par (D 0,9 - D 0,1)/D 0,5 ; la valeur D 0,1 est lorsque 10 % des particules sont en dessous de cette valeur, la valeur D 0,5 est le diamètre médian lorsque 50 % de la distribution sont au-dessus de cette valeur et 50 % sont en dessous de cette valeur et la valeur D 0,9 est lorsque 90 % des particules sont en dessous de cette valeur ;
et un support oralement acceptable.

2. Composition selon la revendication 1, comprenant un agent abrasif supplémentaire.

3. Composition selon l'une quelconque des revendications 1 ou 2, dans laquelle l'oxyde d'aluminium fusionné sphérique est le seul abrasif dans le dentifrice.

4. Composition selon l'une quelconque des revendications 1 à 3, comprenant un phosphate condensé hydrosoluble.

5. Composition selon la revendication 4, dans laquelle le phosphate condensé est un sel de tripolyphosphate de métal alcalin hydrosoluble.

6. Composition selon l'une quelconque des revendications 1 à 5, comprenant un agent désensibilisant.

7. Composition selon l'une quelconque des revendications 1 à 6, comprenant une source d'ions fluorure.
